Europäisches Patentamt

European Patent Office

Office européen des brevets

(19)

(11) Veröffentlichungsnummer: **0 077 807**
**B1**

(12) **EUROPÄISCHE PATENTSCHRIFT**

(45) Veröffentlichungstag der Patentschrift:
**19.11.87**

(21) Anmeldenummer: **82901498.4**

(22) Anmeldetag: **04.05.82**

(86) Internationale Anmeldenummer:
**PCT/DE 82/00101**

(87) Internationale Veröffentlichungsnummer:
**WO 82/03782 (11.11.82** Gazette 82/27)

(51) Int. Cl.⁴: **A 61 N 1/365**

(54) **HERZSCHRITTMACHER.**

(30) Priorität: **04.05.81 DE 3118100**

(43) Veröffentlichungstag der Anmeldung:
**04.05.83 Patentblatt 83/18**

(45) Bekanntmachung des Hinweises auf die Patenterteilung:
**19.11.87 Patentblatt 87/47**

(84) Benannte Vertragsstaaten:
**DE FR GB NL**

(56) Entgegenhaltungen:
**EP - A - 0 016 574**
**FR - A - 2 440 201**
**FR - A - 2 494 119**
**US - A - 3 942 534**
**US - A - 4 280 502**

(73) Patentinhaber: **BIOTRONIK Mess- und Therapiegeräte GmbH & Co Ingenieurbüro Berlin, Sieversufer 8, D-1000 Berlin 47 (DE)**

(72) Erfinder: **NETTELHORST, Herwig, Frhr., v., Pössnecker Strasse 30, D-1000 Berlin 45 (DE)**
Erfinder: **REXHAUSEN, Hermann, Mellinger Strasse 48, D-3200 Hildesheim (DE)**
Erfinder: **SCHALDACH, Max, Turnstrasse 5, D-8520 Erlangen (DE)**
Erfinder: **SCHWEIGGERT, Eugen, Selbitzer Strasse 81a, D-1000 Berlin 22 (DE)**

(74) Vertreter: **Christiansen, Henning, Dipl.-Ing., CHRISTIANSEN & NINNEMANN Dietrich-Schäfer-Weg 21, D-1000 Berlin 41 (DE)**

EP 0 077 807 B1

## Beschreibung

Die Erfindung betrifft einen Herzschrittmacher der im Oberbegriff des Anspruchs 1 angegebenen Art.

Aus der US-A-3 942 534 ist ein künstlicher Herzschrittmacher bekannt, bei dem Tachykardiezustände über im Vorhof abgeleitete Signalimpulse erkannt werden, wobei das Vorliegen des Tachykardiezustands angenommen wird, wenn diese eine vorgegebene Rate überschreiten. Die obere Grenze für die Auffindbarkeit von Tachykardien durch die hier beschriebenen Erkennungsmittel wird gesetzt durch diejenige Folgerate von im Atrium aufgenommenen Impulse, welche bereits die Störerkennungsmittel ansprechen lassen.

Problematisch bei der Erkennung von Tachykardien ist die Tatsache, dass zu denjenigen Zeiten, während der die Eingangsstufen für die Verarbeitung der vom Herzen abgeleiteten Signale durch Austastung abgeschaltet werden, keine sichere Aussage über das Auftreten, den Fortbestand oder die Beendigung eines tachykarden Zustands möglich ist.

Der im Anspruch 1 angegebenen Erfindung liegt die Aufgabe zugrunde, eine Tachykardie-Erkennungsschaltung anzugeben, welche – insbesondere mit einem physiologisch arbeitenden Schrittmachersystem – eine Aussage über das Vorliegen eines Tachykardie-Zustands in der Weise zulässt, dass die abgegebenen Stimulationsimpulse die Patientensicherheit in grösstmöglicher Weise gewährleisten und die Synchronität mit dem Eigenrhythmus der Herzsignale des Patienten erhalten bleibt.

Besonders vorteilhaft ist bei der Erfindung neben dem geringen Bauteileaufwand, dass die Schaltung zur Tachykardie-Erkennung mit einer Schaltung zur Erkennung von Störsignalen unter Übernahme des grössten Teils der Bauelemente kombiniert werden kann und somit auch der Raum- und Energiebedarf gegenüber einem entsprechenden, herkömmlichen Schrittmacherkonzept nur unbedeutend vergrössert ist.

Die Erfindung beruht auf der Erkenntnis, dass beim Einschalten eines das Vorliegen des Tachykardiezustandes festhaltenden Bauelementes die Berücksichtigung der Austastzeiten des Atrium-Eingangsverstärkers im wesentlichen unerheblich ist, weil es dadurch im Mittel höchstens zur Verzögerung der Erkennung dieses Zustandes um ein Tachykardie-Intervall kommt und damit diejenigen Mittel des Schrittmachers, welche eine Betriebszustandsänderung des Schrittmachers in der Richtung bewirken, dass eine Signalaufnahme im Atrium unterbunden wird und gegebenenfalls Massnahmen zur Tachykardiebeendigung ergriffen werden, höchstens verzögert eingeschaltet werden. Der Schrittmacher befindet sich dabei – insbesondere bei Verwendung eines physiologisch reagierenden Schrittmacherkonzepts – noch aufgrund der vom Herzen aufnehmbaren Signale, insbesondere bezüglich seiner Refraktärzeiten in Synchronität mit dem Herzrhythmus des Patienten.

Anders liegen die Verhältnisse jedoch beim Verlassen des durch das Vorliegen der Tachykardie eingeschalteten veränderten Betriebszustands: Wird der während der Tachykardie vorliegende Betriebszustand des Schrittmachers vorzeitig verlassen, ohne dass die Tachykardie tatsächlich beendet wurde, so treten Fehler in der Synchronisation zwischen Herz und Schrittmacher auf, welche eine Stimulation in die vulnerable Phase des Herzens zur Folge haben können. Hier wird durch die Erfindung insoweit Abhilfe geschaffen, dass ein Verlassen des bei einer Tachykardie anzuwendenden Betriebszustands nur dann möglich ist, wenn sichergestellt ist, dass die Herzrate wieder einen normalen Wert angenommen hat. Hierfür ist es erfindungsgemäss Bedingung, dass zwischen dem letzten den Verstärker für im Atrium aufgenommene Signale austastenden Signal und dem darauffolgenden im Vorhof aufgenommenen Signalimpuls mindestens eine Zeitdauer vergangen ist, welche grösser ist als die Zeitdauer, welche der Aufeinanderfolge von tachykarden Impulsen entspricht. Diese Bedingung ist auch dann erfüllt, wenn nach einer entsprechenden Austastzeit zwischen zwei aufeinanderfolgenden aus dem Atrium aufgenommenen Signalen mindestens diese Zeit vergangen ist. Das Verlassen des Tachykardiezustands erfolgt demnach asynchron.

Bei bevorzugten Weiterbildungen der Erfindung wird die Zeitmessung jeweils zwischen dem Austastimpuls aufgrund einer Stimulation im Ventrikel und dem Zeitpunkt, zu dem eine Stimulation im Atrium erfolgen kann, bzw. dem Beginn des mit der Stimulation einhergehenden Austastimpulses, vorgenommen. Bei den Austastsignalen handelt es sich um Impulse, welche auf einen an eine Kammer abgegebenen Stimulationsimpuls hin zur Sperrung der Eingangsmittel erzeugt werden. Die Dauer des Austastimpulses wird günstigerweise dann verlängert, wenn aufgrund einer Extrasystole eine Stimulation im Ventrikel erfolgt, um das Setzen der Mittel zum Festhalten des Vorliegens des Tachykardiezustands durch die retrograde Überleitung des Stimulationsimpulses in das Atrium zu verhindern.

Die Erfindung basiert auf dem Bestreben, eine universelle Schrittmacherstruktur anzugeben, welche Fehlermöglichkeiten bekannter Systeme vermeidet, wobei die Realisierung in einem implantierbaren System möglich sein soll. Das System soll eine weitestgehende Auswertung der aufgrund von vom Herzen abgeleiteten Signalen zur Verfügung stehender Informationen ermöglichen, wobei der Einfluss von Störsignalen gering gehalten ist. Durch die physiologisch korrekte Zuordnung aller die Betriebsarten des Schrittmachers betreffenden Umschaltvorgänge wird eine grosse Patientensicherheit erreicht.

Vorteilhafte Weiterbildungen sind in den Unteransprüchen angegeben und werden einschliesslich der Erfindung anhand des in den Figuren dargestellten Ausführungsbeispiels näher erläutert. Es zeigen:

Figur 1 ein Blockschaltbild der den erfindungsgemässen Schrittmacher bildenden Baugruppen
unter besonderer Berücksichtigung der im Betrieb
auftretenden logischen Verknüpfungen,

Figur 2 ein Zeitdiagramm zur Erläuterung des
Funktionsablaufs bei dem in Fig. 1 dargestellten
Schrittmacher,

Figur 3a Einzelheiten einer zur Erkennung von
Störsignalen dienenden Baugruppe,

Figur 3b Einzelheiten einer zur Erkennung von
Tachykardien dienenden Baugruppe,

Figur 4 Einzelheiten einer zum Einschalten verschiedener Betriebszustände dienenden Baugruppe,

Figur 5 Einzelheiten einer Baugruppe zur
Steuerung der Austastzeiten für die Eingangsstufen in Fig. 1.

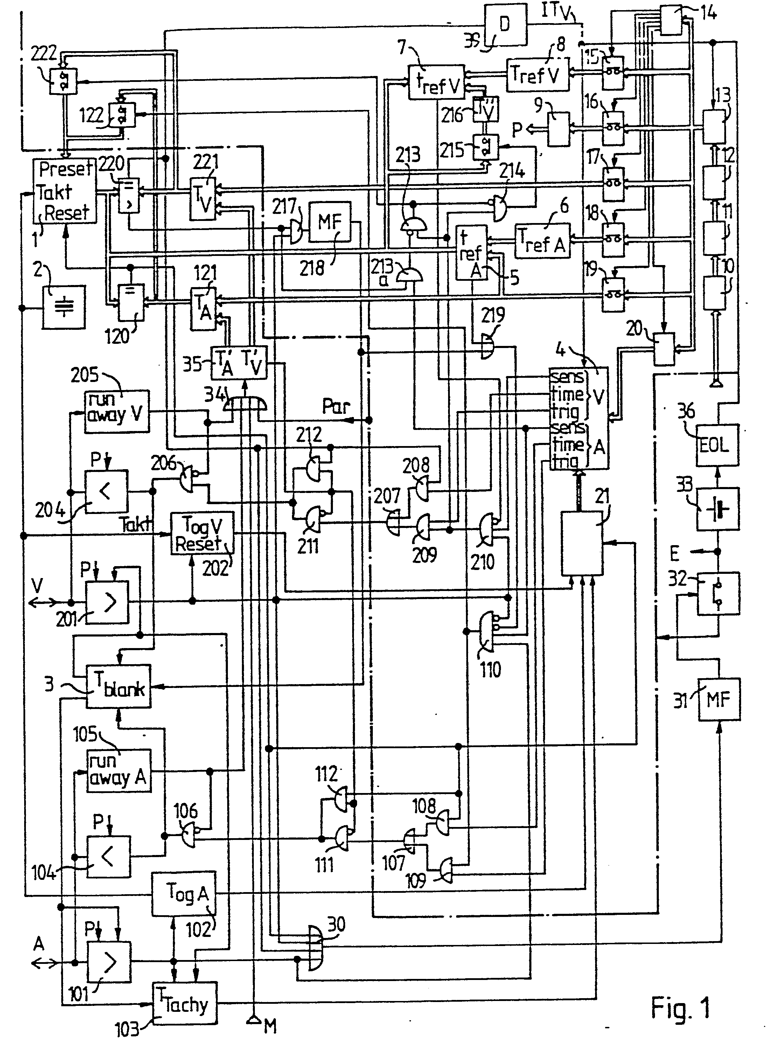

Das in Figur 1 dargestellte in Blockschaltungsform wiedergegebene Prinzipschaltbild des erfindungsgemässen Herzschrittmachers gibt den Signalfluss zwischen den einzelnen Baugruppen
und eine Anzahl von logischen Verknüpfungen
wieder. Der verwendeten Schrittmacherstruktur
liegt das Konzept eines Schrittmachers zugrunde,
das auf grund von Zu- und Abschaltungen von
Signalverbindungen in unterschiedlichen Betriebsarten (Modes) benutzt werden kann, wobei
die Umschaltung zwischen den einzelnen Betriebsarten (die im Folgenden mit den auf dem
Gebiet der Herzschrittmachertechnik eingeführten generischen Codes bezeichnet werden sollen)
programmiert und/oder signalabhängig erfolgen
kann. In seinen komplexeren Modes passt der
Schrittmacher sich dabei auf physiologische Weise dem natürlichen Herzverhalten an.

Die Realisierung des dargestellten Konzepts
kann gleichermassen mittels diskreter oder programmierbarer Logik-Bauelemente erfolgen, wobei im Falle einer hardwaremässigen Lösung die
erforderlichen Verknüpfungen – wie dargestellt –
mittels Logik-Gattern und Speichermitteln wie
Flip-Flops und Latches erfolgen, während beispielsweise bei einer Mikroprozessorlösung die
Logik-Entscheidungen nicht parallel, sondern
nach Programm seriell vorgenommen werden.
RAM-Speicherplätze übernehmen dabei die Funktion der Speichermittel zum Festhalten von für
Betriebszustände charakteristischen Signalen. Einige der dargestellten Funktionsbaugruppen stellen vorteilhafte Weiterbildungen der Erfindung
dar.

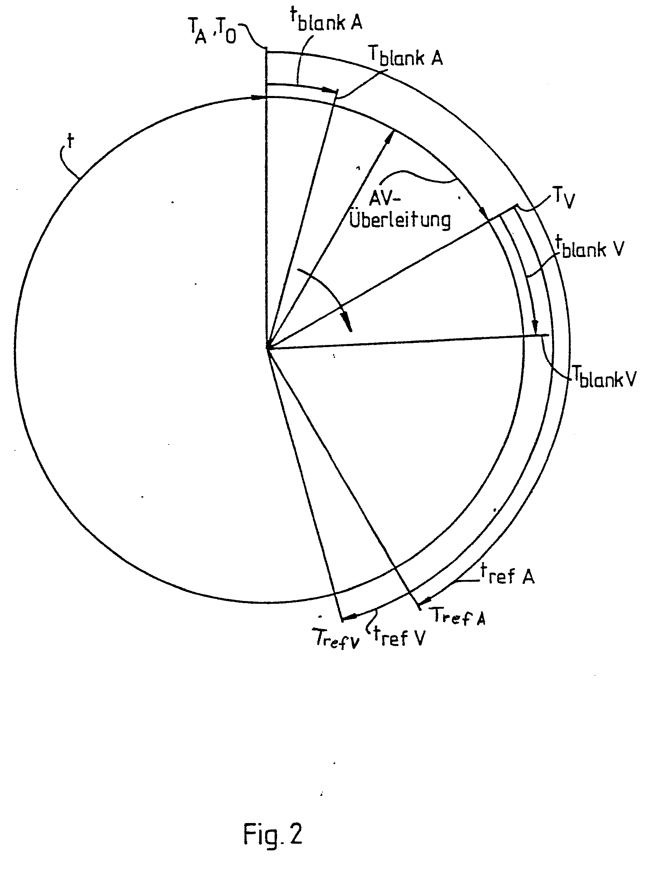

Aus Figur 2 ist der zeitliche Ablauf der Signalverarbeitung und Stimulationsvorgänge ersichtlich, wobei in den übrigen Darstellungen entsprechende Zeitbezeichnungen verwendet wurden, so dass diesbezüglich auf diese Figur stets
Bezug genommen werden kann. In der Darstellung fällt der Anfangszeitpunkt $T_o$ zusammen mit
dem Zeitpunkt $T_A$, der nach einem Umlauf (entsprechend einem Herzzyklus) erreicht wird und
die Aktivität des Atriums charakterisiert. Im Falle
einer Stimulation im Ventrikel oder Atrium folgt
eine Austastzeit $t_{blankA}$ für die im Atrium plazierte
Elektrode, wobei die Dauer dieser Austastzeit jeweils davon abhängig ist, ob im Atrium oder im Ventrikel, d.h. in derselben oder in der jeweils
anderen Kammer stimuliert wurde. Das Ende dieser Austastzeit ist mit $T_{blankA}$ bezeichnet.

Zwischen $T_A$ und $T_V$ befindet sich die AV-Überleitungszeit, wobei $T_V$ den Zeitpunkt der Aktivität
des Ventrikels charakterisiert. An $T_V$ schliesst sich
entsprechend eine Austastzeit $t_{blankV}$ an, welche
im Zeitpunkt $T_{blankV}$ endet. Die Refraktärzeit des
Atriums $t_{refA}$ beginnt mit $T_o$ und endet bei $T_{refA}$,
während die Refraktärzeit des Ventrikels bei $T_V$
beginnt und bei $T_{refV}$ endet. Die Zeiten $T_{og}$ und
$T_{tachy}$ sind aus dem Diagramm gemäss Figur 2
nicht direkt ersichtlich, da diese durch entsprechende Folgeraten der Herzaktionspotentiale
und/oder Störsignale als Triggersignale festgelegt werden.

Bei dem in Figur 1 dargestellten Herzschrittmacher bilden die Anschlüsse A und V Ein- und Ausgänge für Signale, welche vom Herzen aufgenommen werden bzw. in Form von Stimulationsimpulsen an das Herz abgegeben werden. Mit «A» ist
dabei der Anschluss für eine im Atrium und mit
«V» der Anschluss für eine im Ventrikel fixierte
Elektrode bezeichnet.

Die zentrale Steuerung für die zeitliche Zuordnung der abzugebenden Impulse erfolgt über einen Zähler 1, welcher durch von einem Taktgenerator 2 erzeugte Impulse inkrementiert wird und
über einen «Reset»-Eingang in eine Ausgangsstellung zurücksetzbar ist. In Abhängigkeit von
vom Herzen aufgenommenen Signalen ist dieser
Zähler über einen zusätzlichen «Preset»-Eingang
auf beliebige Zählerstände in Vorwärtsrichtung –
entsprechend einer zeitlichen Voreilung – voreinstellbar. Ein Verändern des Zählerstandes in
Rückwärtsrichtung ist nicht notwendig. Nach Erreichen seines Endstandes setzt der nachfolgende Impuls den Zähler auf 0, von wo aus der Zählvorgang fortgesetzt wird. Das Zusammenwirken
des Zählers mit den übrigen in Figur 1 dargestellten Bauelementen wird weiter unten näher beschrieben. (Die Übertragung einzelner digitaler
Signalimpulse oder analoger Signale ist in den
Figuren durch einfache Verbindungen dargestellt,
während doppelte Linien die Übertragung vollständiger Datenwörter symbolisieren.)

Die vom Atrium über eine dort fixierte Elektrode
aufgenommenen Signale gelangen über den Anschluss A an eine Eingangsstufe 101, welche einen Vorverstärker enthält, der über einen Programmiereingang (zugehöriger Pfeil P) in seinem
Verstärkungsfaktor einstellbar ist. Die Eingangsstufe bewirkt gleichzeitig eine Impulsformung in
der Weise, dass die vom Atrium aufgenommenen
Signale in Form von impulsförmigen Logiksignalen in den nachfolgenden Schaltstufen weiterverarbeitet werden können. Die Darstellung der Logikpegel bei dem wiedergegebenen Ausführungsbeispiel erfolgt in positiver Logik, d.h. das Vorhandensein eines Signals wird mit dem H-Zustand
und die Abwesenheit mit dem L-Zustand bezeichnet. Umschaltelemente (Zähler, Mono- und Flip-
Flops) werden durch die ansteigenden Vorderflanken der Signalimpulse aktiviert. Weiterhin weist
die Eingangsstufe 101 Filtermittel auf, mit einer Filtercharakteristik, die sie vorzugsweise für die aufzunehmenden Nutzsignale durchlässig macht.

Die Eingangsstufe 101 wird ausserdem von einer Austastschaltung 3 beeinflusst, welche die Eingangsstufe für vorgegebene Zeiträume $t_{blankA}$ sperrt, wenn ein Stimulationsimpuls an Atrium oder Ventrikel abgegeben wurde. Auf diese Weise wird verhindert, dass ein vom Schrittmacher abgegebener Stimulationsimpuls als herzeigenens Signal verarbeitet wird und das Betriebsverhalten des Schrittmachers fehlerhaft beeinflusst.

Der Eingangsstufe nachgeschaltet ist eine Störerkennungsschaltung 102, welche ein Ausgangssignal abgibt, wenn der Abstand zweier aufeinanderfolgender, von der Eingangsstufe 101 verarbeiteter Signalimpulse einen vorgegebenen zeitlichen Mindestabstand ($T_{og}$), entsprechend einer maximalen Folgefrequenz, unterschreitet. Derartige eine Störung bildende Signale können ihre Ursache sowohl ausserhalb (elektromagnetische Einstreuungen oder Wechselströme) als auch innerhalb des Körpers des Patienten (Elektrodenartefakte) haben. Die Störerkennungsschaltung 102 verhindert im Zusammenwirken mit weiter unten zu beschreibenden Mitteln, dass an der Atriumelektrode aufgenommene Impulse, welche die vorgegebene Frequenz überschreiten, zur Weiterverarbeitung zugelassen werden.

Der Ausgang der Eingangsstufe 101 ist weiterhin mit dem Eingang einer Tachykardieerkennungsstufe 103 verbunden, welche entsprechend ein Signal abgibt, wenn die am Ausgang der Stufe 101 erscheinenden Impulse eine vorgegebene Folgezeit unterschreiten. Die «Tachykardierate» $T_{Tachy}$ ist mit ca. 250 ms grösser als die als Kriterium für das Vorhandensein eines Störsignals festgelegte Zeitdauer $T_{og}$, welche ca. 100 ms beträgt.

Der Anschluss V, an den eine im Ventrikel zu fixierende Elektrode angeschlossen werden kann, ist mit dem Eingang einer weiteren Eingangsstufe 201 verbunden, die in ihren grundsätzlichen Funktionen der Eingangsstufe 101 für vom Atrium aufgenommene Signale entspricht. Der Verstärkungsfaktor ist über einen zusätzlichen Eingang (zugehöriger Pfeil P) einstellbar. Eine separate Störerkennungsschaltung 202 für die von der im Ventrikel fixierten Elektrode aufgenommenen Störsignale gibt ein Ausgangssignal ab, wenn die vom Ventrikel aufgenommenen Signalimpulse eine höhere als eine vorgegebene Frequenz aufweisen. Die Eingangsstufe 201 wird entsprechend durch von der Austaststufe 3 abgegebene Signale zeitweise gesperrt.

Stimulationsimpulse für das Atrium werden mittels einer Impulsformerschaltung 104 erzeugt, wobei die Amplitude der Stimulationsimpulse über einen zusätzlichen Eingang (zugehöriger Pfeil P) einstellbar ist. Die Impulsformerstufe 104 wird durch kurzzeitige Impulse mit logischem H-Pegel angesteuert und enthält auch die zur Heraufsetzung der Eingangsimpulse auf den zur Stimulation notwendigen Pegel erforderlichen Verstärkungsmittel.

Die von der Impulsformerstufe 104 abgegebenen Impulse gelangen einerseits zum Anschluss A und andererseits zu einer Run-away-Schutzschaltung 105, welche in dem dargestellten Ausführungsbeispiel durch ein Monoflop gebildet wird, dessen Impulsdauer auf die höchste zulässige Stimulationsrate im Atrium abgestimmt ist. Auf einen Ausgangsimpuls von der Impulsformerstufe 104 hin gibt die Schutzschaltung 105 einen Impuls vorgegebener Breite an den invertierenden Eingang eines UND-Gatters 106 ab, welches daraufhin für die Dauer des letztgenannten Impulses hin für an seinen anderen Eingang gelangende Signalimpulse gesperrt ist.

Eine Impulsformerstufe 204 für zum Stimulieren des Ventrikels bestimmte Impulse ist ebenfalls über einen weiteren Eingang (zugehöriger Pfeil P) bezüglich der Amplitude der abgegebenen Impulse beeinflussbar. Eine separate Run-away-Schutzschaltung 205 für den Ventrikel sperrt ein UND-Gatter 206 über dessen invertierenden Eingang, wenn die Folgezeit der den Ventrikel stimulierenden Impulse den vorgegebenen Wert unterschreitet.

Die Zeiten, zu denen Stimulationsimpulse abgegeben werden können (mit $T_A$ und $T_V$ bezeichnet), bestimmt der Zähler 1, wobei jeweils für Atrium und Ventrikel separate Vergleicherschaltungen 120 bzw. 220 vorgesehen sind, welche einen Impuls am Ausgang «=» erzeugen, wenn der Zählerstand des Zählers 1 mit einem vorgegebenen Zählerstand in einem Speicher 121 für den Stimulationszeitpunkt des Atriums bzw. einem entsprechenden Speicher 221 für den Ventrikel übereinstimmt. Die Vergleicherschaltung 220 gibt über einen zusätzlichen Ausgang «>» ein Signal ab, wenn der Zählerstand des Zählers grösser ist als der in dem Speicher 221 festgehaltene Wert für $T_V$. Die genannten Zeitmarken $T_A$ und $T_V$ bilden Bezugszeiten für den Betrieb des Schrittmachers und beenden im Demand-Betrieb die sogenannten «Escape-Intervalle» innerhalb denen eine entsprechende Eigenaktion des Herzens die Erzeugung eines Stimulationsimpulses durch den Schrittmacher verhindert.

Die Zeiten $T_A$ und $T_V$ für Atrium und Ventrikel in den Speichern 121 bzw. 221 werden in Form von Zahlenwerten programmiert, welche diejenigen Zählerstände repräsentieren, die der Zähler 1 im Stimulationszeitpunkt erreicht haben muss. Die Zeitzuordnung zu den Zählerständen erfolgt in der Weise, dass die Stimulationszeitpunkte mit einem ausreichend feinen Zeitraster einstellbar sind. Bei einem Zählertakt von beispielsweise 1 kHz weist das zur Verfügung stehende Zeitraster eine Teilung in Millisekunden auf. Die mittels der Speicher 121 bzw. 221 vorgebbaren Zählerstände sind durch weiter unten darzustellende Mittel veränderbar.

Die Eingangsimpulse für die Ausgangsstufe 104, welche das UND-Gatter 106 passieren, stammen vom Ausgang eines ODER-Gatters 107, an dessen Eingänge alternativ die über die Abgabe eines Stimulationsimpulses an das Atrium entscheidenden Signale gelangen. Einer der Eingänge des ODER-Gatters 107 ist mit dem Ausgang eines UND-Gatters 108 verbunden, das ein Ausgangssignal abgibt, wenn die Vergleicherschaltung 120 das Erreichen des Zeitpunkes $T_A$ angibt und damit anzeigt, dass im Demand-Zustand der Vorhofstimulation ein Zeitpunkt erreicht wurde, bei dem wegen fehlender eigener Aktivität des Herzens ein Stimulationsimpuls erforderlich ist. Das UND-Gatter 108 wird über seinen weiteren Eingang mittels eines Signals durchgeschaltet, welches von einem Ausgang «time$_A$» des Betriebszustandsregister 4 abgegeben wird, wenn eine Impulsabgabe nach Zeitablauf erwünscht ist. Bei freigegebener Signalaufnahme (sens$_V$ bei Block 4) für die entsprechende Kammer erfolgt damit ein «Demand»-Betrieb mit Inhibierung durch herzeigene Impulse, während bei gesperrter Signalaufnahme eine Stimulation zu festen Zeiten erfolgt.

Ein weiteres UND-Gatter 109 wird über einen entsprechenden Ausgang «trig$_A$» der Schaltung 4 bei durch Herzeigenaktionen im Vorhof getriggerter Signalabgabe an den Vorhof aktiviert. Das Signal zum synchronen Auslösen des Stimulationsimpulses im Vorhof gelangt an den Eingang des UND-Gatters 109 vom Ausgang eines weiteren UND-Gatters 110, das seinerseits durch ein von einem Ausgang «sens$_A$» der Schaltung 4 an seinen Eingang gelangendes Signal aktiviert wird, wenn die Aufnahme von Signalen aus dem Vorhof bei der gewählten Betriebsweise des Schrittmachers vorgesehen ist.

Es ist ersichtlich, dass für den durch herzeigene Signale getriggerten Betrieb diese Freigabe der Signalaufnahme Voraussetzung ist. An das UND-Gatter 110 gelangt nicht nur das Ausgangssignal der Eingangsstufe 101, sondern an dessen weiteren invertierenden Eingang auch das Ausgangssignal einer Refraktärstufe für den Vorhof 5, welche ein Signal abgibt, wenn für einen Zeitraum $t_{refA}$, der mit dem Erreichen des dem Zeitpunkt $T_A$ entsprechenden Zählerstand durch den Zähler 1 beginnt, die Verarbeitung der im Atrium aufgenommenen Signale zur Auswertung für die Abgabe von Stimulationsimpulsen gesperrt ist. Ein der Refraktärzeit $T_{refA}$ für das Atrium entsprechender Zahlenwert ist in einem Speicher 6 festgehalten und über weiter unten dargestellte Programmiermittel durch äussere Signale veränderbar.

Ein ODER-Gatter 207 und UND-Gatter 208 bis 210 bilden die entsprechenden, über die Abgabe von Stimulationsimpulsen an den Ventrikel bzw. über die Signalaufnahme aus dem Ventrikel entscheidenden Logik-Gatter. Das gewünschte Betriebsverhalten des Schrittmachers lässt sich ebenfalls über drei Ausgänge «sens$_V$», «time$_V$» und «trig$_V$» der Schaltung 4 bestimmen, wobei das an den weiteren Eingang des UND-Gatters 208 gelangende Signal die Stimulation freigibt, wenn der Stand des Zählers 1 dem im Speicher 221 festgehaltenen, dem Zeitpunkt $T_V$ zugeordneten Zählerstand entspricht und somit zu diesem Zeitpunkt ein Ausgangssignal des Vergleichers 220 an das UND-Gatter 208 ausgegeben wird.

Die synchrone Stimulation wird durch das UND-Gatter 209 ausgelöst, dem das Ausgangssignal des UND-Gatters 210, welches die vom Ventrikel aufgenommenen Signalimpulse weiterleitet, zugeführt wird. Das UND-Gatter 210 ist über seinen invertierenden Eingang während der Ventrikel-Refraktärzeit $t_{refV}$ gesperrt, welche mittels eines Vergleichers 7 festgelegt wird, wobei der erreichte Zählwert des Zählers 1 mit dem in dem Speicher 8 festgehaltenen, die Zeitdauer $T_{refV}$ repräsentierenden Zählwert verglichen wird und zu dem Inhalt des Speichers 8 derjenige des Speichers 216, dessen Inhalt $T_V''$ im Normalfall der Zeit $T_V$ entspricht, hinzuaddiert wird. (Unterschiede zwischen der Funktion der Speicher 6 für die Refraktärzeit des Atriums und 7 für die Refraktärzeit des Ventrikels sind aus der folgenden Beschreibung ersichtlich.) Der Inhalt des Speichers 8 ist wie derjenige des Speichers 6 über äussere Programmiermittel veränderbar.

Die Ausgangssignale der UND-Gatter 110 bzw. 210 beeinflussen des weiteren Schalter 122 und 222, mittels denen der Zähler 1 über seinen Preset-Eingang auf die in den Speichern 121 (für den Wert $T_A$) bzw. 221 (für den Wert $T_V$) vorliegenden Werte voreinstellbar ist. Damit wird der Zähler 1 beim Erscheinen eines intrakardialen Signals im Atrium auf den der Zeit $T_A$ bzw. beim Erscheinen eines Signals im Ventrikel auf den der Zeit $T_V$ entsprechenden Zahlenwert vorangesetzt. Auf diese Weise wird der Schrittmacher mit dem Herzverhalten synchronisiert, wenn die UND-Gatter 110 bzw. 210 über ihre entsprechenden Signaleingänge für die Signalaufnahme vom Herzen freigegeben sind. Sind die zuletzt genannten Gatter gesperrt, so erfolgt die Stimulation gegebenenfalls asynchron.

Die Inhalte der Speicher 4, 6 und 8 sowie der eines weiteren Speichers 9, welcher die programmierbaren Verstärkungsfaktoren bzw. Ausgangssignalamplituden repräsentierenden Daten für die Stufen 101 und 201 sowie 104 und 204 (zugeordnete Pfeile P) enthält, sind über die blockschaltungsmässig dargestellten Programmiermittel zu beeinflussen, welche nachfolgend kurz beschrieben werden sollen:

Die von ausserhalb des Schrittmachers über einen geeigneten Nachrichtensignalträger (radiofrequente, optische oder sonstige Signale) in Form von Impulsen aufmodulierten, von einem entsprechenden – nicht dargestellten – Sender abgegebenen Signale, gelangen zu einem Empfänger 10, der die verstärkten Signale einem Dekoder 11 zuleitet, mit dessen Hilfe die zu übertragene ursprüngliche Programmierimpulsfolge wieder hergestellt wird. In einer Prüfstufe 12 wird mittels zusätzlich übertragener redundanter Signale festgestellt, ob die übertragene Information vollständig ist und letztere zutreffendenfalls an einen Steuerbaustein 13 übermittelt, durch den mittels einer ersten Teilinformation eine Auswahlschaltung 14 entsprechend aktiviert wird.

Durch das Ausgangssignal der Schaltung 14 wird eine der Schaltstufen 15 bis 20 betätigt, so dass die zweite übertragene Teilinformation in den mit der aktivierten Stufe der Stufen 15 bis 20 verbundenen Speicher gelangt und dort festgehalten wird. Die bereits erwähnten Speicherstufen 4, 6, 8, 9 sowie 121 und 222 halten die für den Betrieb des Schrittmachers wesentlichen veränderbaren Daten fest, wie sie in der vorangehenden Beschreibung aufgeführt wurden.

Der Speicher 4 ist von zusätzlichen Signalen beeinflussbar, welche aus der Signalverarbeitungsstufe 21 stammen, und eine Umschaltung der Betriebszustände in Abhängigkeit von vom Schrittmacher selbst aus dem Herzen aufgenommenen Signalen bewirkt, wobei die Eingangssignale der Stufe 21 von den Stufen 102 und 202 stammen und im Atrium bzw. im Ventrikel aufgenommene Störsignale repräsentieren, sowie von der Tachykardiestufe 103, welche ein Ausgangssignal abgibt, wenn der Tachykardiezustand festgestellt wurde.

Die von der strichpunktierten Linie umgebenen Bauelemente zur Signalverarbeitung und -speicherung sind jeweils nur zeitweise aktiviert, da die zum Betrieb des Schrittmachers notwendigen logischen Operationen mittels moderner mikroelektronischer Bauelemente in sehr kurzen Zeiten ausgeführt werden können, so dass deren permanenter Betrieb einen unnötigen Energieverbrauch zur Folge haben würde. Die ausserhalb der genannten Linie befindlichen Bauelemente, die entweder direkt mit den im Herzen fixierten Elektroden verbunden sind und damit die Signalaufnahme vom Herzen kontrollieren, bzw. diejenigen Mittel, welche die Zeitgeber- und Zeitvergleichermittel bilden, sind dauernd aktiviert, da durch letztere bestimmt wird, zu welchen Zeitpunkten logische Operationen auszuführen sind.

Die Aktivierung der innerhalb der strichpunktierten Linie befindlichen Bauelemente erfolgt durch die von den Vergleichermitteln 120 bzw. 220 abgegebenen Signale, wenn ein vorgegebener Zeitpunkt erreicht wurde, oder aber durch die Ausgangssignale der Eingangsstufen 101 bzw. 201, wenn nämlich vom Herzen (ausserhalb der jeweiligen Refraktärzeit) ein Signal aufgenommen wurde. Die Zusammenfassung der genannten Signale erfolgt über ein ODER-Gatter 30, welches über ein Monoflop 31 einen Schalter 32 betätigt, der die in der gewählten Darstellung innerhalb der strichpunktierten Linie angeordneten Bauelemente mit einer Energiequelle 33 verbindet, wobei dieses «Verbinden» mit einer Energiequelle in jedem Heraufsetzen der Energieversorgung, also auch in einem Umschalten der betreffenden Bauelemente von einem Bereitschafts-(Stand-by-)Zustand in einen Betriebszustand bestehen kann.

Die Impulsdauer des Monoflops 31 ist dabei so bemessen, dass zum Ausführen der notwendigen Schaltvorgänge ein ausreichend langer Zeitraum zur Verfügung steht. Diskrete Logikbauelemente lassen sich energiesparend bei derart herabgesetzter Versorgungsspannung betreiben, dass zwar keine Veränderungen vorgenommen werden können, gespeicherte Signalzustände jedoch erhalten bleiben. Diese Bauelemente dienen der nachfolgenden Signalverarbeitung in dem Sinne, dass sie festlegen, ob auf ein Eingangssignal oder beim Erreichen eines vorgegebenen Zeitpunkts ein Stimulationsimpuls abgegeben werden soll.

Der von der Energiequelle 33, die im dargestellten Ausführungsbeispiel von einer Batterie gebildet wird, ausgehende Pfeil E deutet an, dass die ausserhalb der strichpunktierten Linie angeordneten Bauelemente direkt von der Energiequelle 33 ohne Zwischenschaltung des Schaltelementes 32 versorgt werden.

Mit der dargestellten Anordnung sind die Betriebsfunktionen üblicher Schrittmachertypen mit synchroner oder asynchroner Stimulation im Atrium und/oder Ventrikel erzeugbar, wobei je nach Zustand der Schaltmittel 4 und Freigabe der entsprechenden Stimulationssignale bzw. Signalaufnahmemöglichkeit über die UND-Gatter 108 bis 110 bzw. 208 bis 210 auch jene Schrittmacherbetriebsarten höherer Ordnung möglich sind, wie sie in Form einer Übersicht beispielsweise in dem Aufsatz «Physiological Cardiac Pacing», R. Sutton, J. Perrins und P. Citron in «PACE»; Vol. 3, März–April 1980, S. 207 bis 219 wiedergegeben sind.

Das grundsätzliche Stimulationsverhalten der verschiedenen Schrittmacherbetriebsweisen bei verschiedenartigen Herzsignalen ist ebenfalls in der vorgenannten Literaturstelle angegeben.

Weitere Einzelheiten zu den in der nachfolgenden Beschreibung dargestellten Schaltungsteilen können den am gleichen Tage eingereichten, diese Schaltungsteile wegen der damit verbundenen Neuerungen näher beschreibenden Patentanmeldungen WO 82/03780, WO 82/03781 und WO 82/03783 bis WO 82/03786 derselben Anmelderin entnommen werden.

Durch die funktionsmässige Trennung des komplexen, nur zeitweise aktivierten Logiksystems von den permanent betriebsbereiten Bauelementen, ist es in vorteilhafter Weise möglich, für den Fall des Ausfalls des umfangreichen Logikteils ein einfaches Ersatzsystem vorzusehen, durch welches ein Notbetrieb aufrecht erhalten werden kann. Obgleich zwar einerseits die Sicherheit einzelner Bauelemente gegen Ausfall in der Vergangenheit wesentlich gesteigert werden konnte, wird andererseits angestrebt, die Informationsverarbeitung in einem künstlichen Herzschrittmacher möglichst allen therapeutischen Anforderungen gerecht werden zu lassen, um dem Patienten eine optimale Stimulationstherapie zukommen zu lassen, und damit die erreichte Erhöhung der Zuverlässigkeit zum Teil wieder kompensiert.

Das dargestellte Konzept gestattet es, die Vorteile eines einfachen, höchst betriebssicheren Systems mit einem solchen höherer Verarbeitungsfähigkeit derart zu verbinden, dass bei Ausfall des komplexen Datenverarbeitungssystems ein einfaches Ersatzsystem bereitsteht, welches in der Lage ist, die Grundfunktionen des Schrittmachers unter nahezu allen Umständen für einen nur durch die Betriebsdauer der Energiequellen begrenzten Zeitraum sicherzustellen.

Diese Ersatzschaltung kann prinzipiell durch verschiedenartige Signale aktiviert werden. Eines dieser Signale ist ein von der komplexen Logikschaltung ausgehendes Signal «Par», das bei einer mittels eines Mikroprozessors realisierten Logikschaltung dann erscheint, wenn innerhalb der durchgeführten Operationen ein Plausibilitätsfehler ermittelt wurde, wie er beispielsweise durch eine Paritätsüberprüfung mit Hilfe von redundanten Signalen in bekannter Weise erzeugt werden kann. Entsprechende Signale lassen sich auch aus mit diskreten Logikbauelementen arbeitenden Schaltungen gewinnen, wenn hier zusätzliche Elemente verwendet werden, welche unzulässige Betriebszustände, beispielsweise in Form von Signalen, erkennen, die bei korrekt arbeitenden Logikmitteln beispielsweise nicht gleichzeitig oder aber nicht mit einer oberhalb oder unterhalb einer vorgegebenen Grenzfrequenz liegenden Rate etc. erscheinen dürfen. Derartige ein Fehlverhalten aufzeigenden Signale werden auch von den Ausgängen der Run-away-Schutzschaltungen 105 und 205 gewonnen und über ein ODER-Gatter 34 zusammengefasst. Bei der dargestellten Anordnung kann es theoretisch in dem Fall zu einem Ansprechen der Run-away-Schutzschaltungen bei ordnungsgemäss arbeitenden Taktgenerator 2 kommen, wenn in die Speicher 121 oder 221 durch einen Fehler in dem durch die strichpunktierte Linie umrandeten Teil der Logikschaltungen fehlerhafte Zahlenwerte eingelesen wurden.

Dem ODER-Gatter 34 wird ein weiteres Signal M zugeführt, welches von einem Reed-Schalter stammt, der in der Zeichnung nicht dargestellt ist. Auf diese Weise ist es möglich, mittels eines von aussen einwirkenden Magnetfeldes den Ersatzbetriebszustand entsprechend der «Magnetfrequenz» der bekannten Schrittmacher einzustellen.

Der Ausgang des ODER-Gatters 34 aktiviert eine Speicherschaltung 35, welche Zeitpunkten $T_A'$ und $T_V'$ entsprechende Zahlenwerte enthält, die bei Aktivierung dieser Speicherschaltung 35 mit Vorrang in die Speicher 121 bzw. 221 eingelesen werden. Gleichzeitig wird von der Schaltung 35 ein Ausgangssignal abgegeben, welches über invertierende Eingänge von UND-Gattern 111 und 211, die zwischen die Gatter 107 und 106 bzw. 207 und 206 eingeschaltet sind und diesen Signalweg sperren, während über die somit aktivierten UND-Gatter 112 und 212 eine direkte Verbindung zu den Ausgängen der Vergleicher 120 und 220 hergestellt wird.

Damit ist das im «D00»-Betrieb arbeitende Ersatzsystem nicht mehr von der Signalverarbeitung der innerhalb der strichpunktierten Linie befindlichen Elemente der Schaltung abhängig. Die Werte $T_A'$ und $T_V'$ werden dabei so gewählt, dass sich eine für die Frequenzen annehmbare Ersatz-Stimulationsfrequenz ergibt. Die Betriebsart «D00» bezieht sich auf den Anschluss zweier Elektroden. Ist jeweils nur eine der beiden Elektroden angeschlossen, so arbeitet der Schrittmacher im Zustand «A00» oder «V00» und gibt damit entsprechend der gewählten Elektrodenkonfiguration ebenfalls die zur Aufrechterhaltung der vitalen Funktionen notwendigen Stimulationsimpulse ab.

Mittels zweier in die Aktivierungsleitung des Schalters 222 eingefügter UND-Gatter 213 und 213a wird eine Synchronisation des Zählers 1 (und damit der Zeitsteuerung des Schrittmachers) auf vom Ventrikel erscheinende Signale für die Zeit der Überleitung vom Atrium zum Ventrikel verhindert (AV-Überleitung zwischen $T_A$ und $T_V$ in Figur 2), wenn gleichzeitig eine Signalaufnahme im Atrium möglich ist. Die entsprechenden Zeitmarken beziehen sich (abhängig von der Betriebsart des Schrittmachers) auf Zeiträume zwischen herzeigenen oder stimulierten Signalen. Dem UND-Gatter 213a werden das Signal sens$_A$ des Speichers 4 und das Signal «>» des Vergleichers 220 an seinen Eingängen zugeführt. Das Ausgangssignal des UND-Gatters 213a sperrt gegebenenfalls das UND-Gatter 213 über dessen invertierenden Eingang. Der weitere Eingang des UND-Gatters ist mit den die Signalaufnahme für das Atrium am UND-Gatter 110 freigebenden Signalausgang des Speichers 4 verbunden. Damit wird erreicht, dass eine Synchronisation bei regulärer Ventrikeltätigkeit stets nur mit $T_A$ erfolgt und somit das Atrium bei AV-sequentieller Stimulation die Führung behält bzw. bei fehlender Signalaufnahme aus dem Atrium der Abstand aufeinanderfolgender Zeitpunkte $T_A$ konstant bleibt.

Der hier beschriebene Schrittmacher stellt damit die physiologisch richtige Synchronisation mit dem Atrium sicher, wenn dort ungestörte Signale zur Verfügung stehen. Ist die Signalaufnahme im Atrium gestört, so erfolgt – entsprechend den vorgesehenen Möglichkeiten der Betriebszustandsänderungen, die weiter unten beschrieben werden – eine Umschaltung in einen eine Signalaufnahme im Ventrikel ermöglichenden Betriebszustand, so dass auch in diesem Fall eine korrekte Stimulation gewährleistet ist.

Obgleich bei freigegebener Signalaufnahme im Atrium für die Zeit t zwischen $T_A$ und $T_V$ keine Synchronisation des Zählers 1 durch im Ventrikel aufgenommene Signale erfolgt, wird die Refraktärzeit des Ventrikels dadurch in physiologisch sinnvoller Weise gesetzt, dass diese sich trotz fehlender Synchronisation des Zählers 1 auf die Zeiten bezieht, zu denen die Herzaktion im Ventrikel festgestellt wurde, was bei dem dargestellten Ausführungsbeispiel dadurch realisiert ist, dass das Ausgangssignal des UND-Gatters 210 über ein UND-Gatter 214 und ein Schaltelement 215 einen Speicher 216 mit einem zu diesem Zeitpunkt im Zähler 1 enthaltenen Zahlenwert lädt, so dass dieser Zeitpunkt $T_V''$ als Bezugszeitpunkt jetzt für die Ermittlung der Refraktärzeit $T_{refV}$ durch den Vergleicher 7 dienen kann. Erfolgt eine Signalaufnahme ausschliesslich im Ventrikel, so ist eine Synchronisation des Zählers durch das Schaltelement 222 im Zeitpunkt $T_V$ freigegeben, wodurch mittels des UND-Gatters 214 in den Speicher 216 dann derjenige Zahlenwert für $T_V$ übertragen wird, welcher auch im Speicher 221 vorhanden ist.

Nach Ablauf der Refraktärzeit für aus dem Ventrikel aufgenommene Signale erscheinende Impulse, welche von Extrasystolen herrühren, synchronisieren den Zeitzähler 1 des Schrittmachers, was bei dem dargestellten Ausführungsbeispiel dadurch erreicht wird, dass der Zähler 1 durch die Schaltermittel 222 auf den Wert $T_V$, der im Speicher 221 vorhanden ist, gesetzt wird. Darüberhinaus muss auch sichergestellt sein, dass retrograd übergeleitete Erregungen nach Extrasystolen keine Synchronisation des Schrittmachers bewirken. Dies wird dadurch erreicht, dass nach Erkennen der Extrasystole (Ventrikelaktivität vor dem Zeitpunkt $T_o$) die Atriumrefraktärzeit $t_{refA}$ für einen Zyklus auf 400 ms gesetzt wird, so dass für einen ausreichend langen Zeitraum (entsprechend der retrograden Überleitungszeit) – ausgehend von der Zeitmarke $T_V$ das Atrium für eine Signalaufnahme gesperrt ist.

Die Sperrung derartiger retrograder Überleitungen ist deshalb von besonderer Bedeutung, weil ein vom Schrittmacher auf das Signal im Atrium hin mit der vorgegebenen AV-Überleitungszeit abgegebener Stimulationsimpuls im Ventrikel eine Stimulation in der vulnerablen Phase zur Folge hätte.

Um hier den notwendigen Schutz zu bewirken, werden unmittelbar auf eine Extrasystole hin die Signalaufnahmemittel für Vorhof und Ventrikel für einen vorgegebenen Zeitraum refraktär geschaltet. Und zwar wird bei einem Eingangsimpuls am UND-Gatter 217 vom Ausgang der Eingangsstufe 201 für den Ventrikel, der erscheint, wenn auch der Vergleicher 220 am Ausgang «>» ein Signal abgibt, das an den zweiten Eingang des UND-Gatters 217 gelangt, ein Monoflop 218 gestartet. Dieses Monoflop gibt daraufhin einen Ausgangsimpuls von ca. 400 ms Dauer ab, welcher an einen Eingang des ODER-Gatters 219 gelangt, dessen zweiter Eingang mit dem Ausgang des Vergleichers 5 für die Refraktärzeit $T_{refA}$ verbunden ist, und dessen Ausgangssignal zu einem der Eingänge des UND-Gatters 110 übertragen wird.

Auf diese Weise ist sichergestellt, dass bei jeder nach der Zeit $T_V$ festgestellten Kammerkontraktion die Refraktärzeit für das Atrium neu gestartet und für die Zeitdauer des von dem Monoflop 218 abgegebenen Impulses (vorzugsweise 400 ms) aufrechterhalten wird, so dass eine Synchronisation des Schrittmachers durch retrograde Überleitungen verhindert ist. (Wie weiter unten näher erläutert ist, sind für diesen Zeitraum auch die Stör- und Tachykardieerkennungsmittel gesperrt, da diese bei einer retrograden Überleitung nicht ansprechen sollten. Die erforderliche Signalverbindung wird durch die Leitung vom Ausgang des Monoflops 218 zum Block 3 gebildet.) Ein ODER-Gatter sperrt auch den Ventrikel, wobei bevorzugt mittels – nicht dargestellter Schaltmittel – statt der Impulszeit des Monoflops 218 auch die in Block 7 enthaltene Zeit heranziehbar ist.

Für den Fall, dass gleichzeitig Signale im Atrium und Ventrikel aufgenommen werden, hat der Ventrikel Vorrang. Damit ist gewährleistet, dass die Sicherheit gegen schädliche Stimulation aufgrund unerwünschter Überleitungen auch bei nicht eindeutiger Signalerkennung zunächst einmal gegeben ist.

Gemäss einer bevorzugten Ausführung der Erfindung werden alle Umschaltungen (Programmänderungen, Betriebsartänderungen) zu Zeitpunkten vorgenommen, welche den Betriebsablauf nicht stören, das sind solche Zeitpunkte, zu denen keine Stimulation bewirkt werden kann und auch kein Eingangssignal erwartet wird, dessen Signalaufnahme wiederum von den eingestellten Betriebsparametern abhängig sein könnte. Ein derartiger Zeitpunkt ist derjenige auf $T_V$ folgende Zeitbereich, in dem beide Eingangsstufen refraktär sind (vergleiche Fig. 2, aus der die Zeiten des Herzzyklus im Hinblick auf den Betrieb des Schrittmachers in ihrer grundsätzlichen Verteilung ersichtlich sind).

Der mit $T_V$ beginnende Zeitraum ermöglicht dabei die für den Betrieb des Schrittmachers notwendigen Umschaltungen in einer Weise, welche die physiologischen Randbedingungen der Stimulation am wenigsten beeinträchtigen, da Umschaltungen zu dieser Zeit in eine natürliche Ruheperiode des Herzens fallen. Im Zeitpunkt $T_V$ finden auch solche Überprüfungen statt, die regelmässig durchgeführt werden müssen, wie die Bestimmung des Betriebszustands der Energiequelle (EOL-Block 36).

Der Zeitpunkt $T_V$ wird bei normalem Herzverhalten stets durchlaufen und wäre an sich ausreichend beispielsweise die Übernahme der geänderten Betriebsparameter (entsprechender Pfeil an Speicherblock 4) oder die Änderung der Programmierung aufgrund der von aussen her übertragenen entsprechenden Signale zu diesem Zeitpunkt zu bewirken (Pfeil nach Block 13).

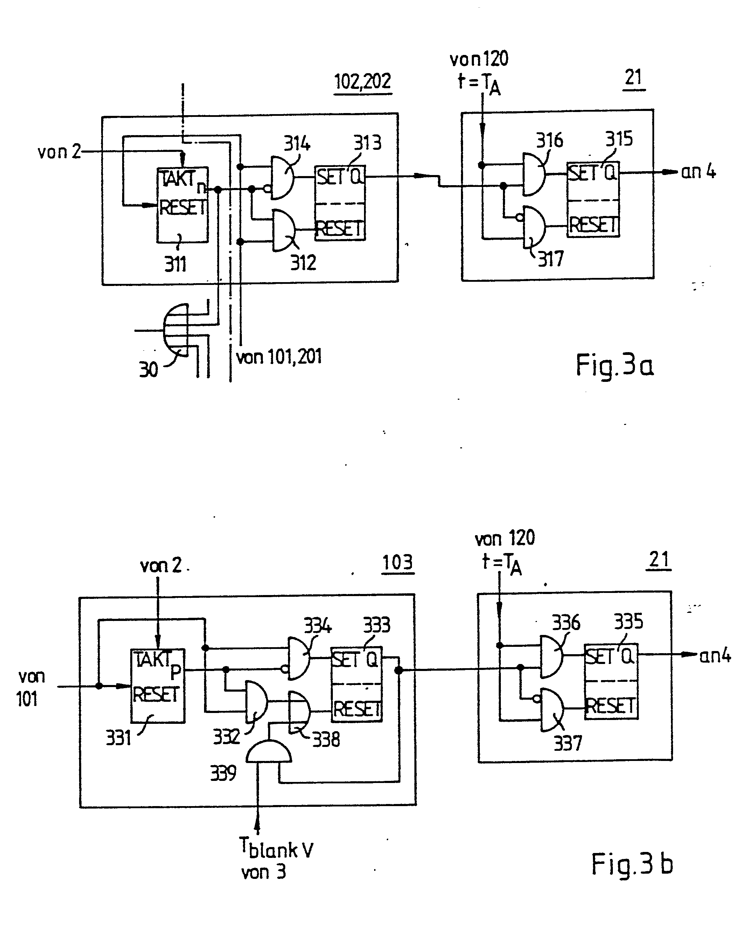

Die in Figur 1 mit den Blöcken 21, 102, 103, 202 dargestellten Schaltungen zur Ermittlung von Störsignalen im Vorhof oder Ventrikel bzw. zur Feststellung des Tachykardiezustandes sind in den Figuren 3a und b im einzelnen dargestellt. Dabei zeigt Figur 3a zum einen (in ihrem linken Bereich) die Detailschaltung der Bausteine 102 bzw. 202 gemäss Figur 1 und in ihrem rechten Teil eine Schaltung, wie sie einen Teil des Schaltungsblocks 21 in Figur 1 bildet – und zwar ist dieser Teil für die Verarbeitung der Eingangssignale von Atrium und Ventrikel je einmal vorhanden. Ein Verzögerungsglied 39 bewirkt, dass eine Umprogrammierung des Schrittmachers im Zeitpunkt $T_V$ erst dann erfolgt, wenn alle anderen bei dieser Zeitmarke auszuführenden Funktionen ausgeführt sind.

Die an den Eingang der Schaltung in Figur 3a gelangenden, von den Eingangsstufen 101 bzw. 201 in Figur 1 stammenden Impulse setzen einen Zähler 311 zurück, welcher durch Taktsignale des Taktgebers 2 (Figur 1) angesteuert wird. Erreicht der Zähler 311 einen Zählerstand n, so gibt er ein Ausgangssignal ab. Die Zahl n ist dabei so gewählt, dass die durch den Zählerstand n erzeugte Frequenzteilung des Eingangstaktes eine Ausgangsfrequenz erzeugen würde, welche gleich der oberen Grenzfrequenz der vom Schrittmacher zugelassenen Eingangssignale ist. Eingangsimpulse, welche eine höhere Rate aufweisen, erzeugen am Ausgang der Blöcke 102 bzw. 202 einen konstanten Pegel, der den Störzustand anzeigt.

Durch das Ausgangssignal n des Zählers 311 wird über ein UND-Gatter 312 ein nachgeschaltetes Flip-Flop 313 zurückgesetzt, wenn an dem anderen Eingang des UND-Gatters 312 der Impuls erscheint, welcher den Zähler 311 zurücksetzt. (Dabei wird davon ausgegangen, dass der Zähler nicht über den Stand n hinauszählt bzw. das Signal am Ausgang n beibehält, solange er nicht zurückgesetzt wird. Wird der Zählerstand n nicht erreicht, wenn der nachfolgende Impuls an dessen Eingang den Zähler zurücksetzt, so wird über ein weiteres UND-Gatter 314 das Flip-Flop 313 gesetzt, wobei der Ausgang «n» des Zählers mit einem invertierenden Eingang des UND-Gatters 314 verbunden ist. Eingangsimpulse, welche die Grenzrate zu Störsignalen überschreiten, setzen demnach das Flip-Flop 313, das vom nächsten Impuls, der mit einem grösseren Zeitabstand als es der Störrate entspricht erscheint, zurückgesetzt wird.

Der Schaltzustand des Flip-Flops 313 wird in ein Flip-Flop 315 durch einen zum Zeitpunkt $t = T_A$ erscheinenden Impuls übertragen, wobei das Ausgangssignal des Flip-Flops 313 an den Eingang eines UND-Gatters 316 und den invertierenden Eingang eines UND-Gatters 317 gelangt, welche mit dem Setz- bzw. Rücksetzeingang des Flip-Flops 315 verbunden sind. Während also das Auftreten von Störsignalen fortgesetzt ermittelt wird, erfolgt eine Übernahme in das Flip-Flop 315 zur weiteren Verarbeitung jeweils nur zum Zeitpunkt $T_A$. Die Übernahme zum Zeitpunkt $T_A$ garantiert, dass die Störung richtig erkannt wurde und somit eine durch Blankingperioden verursachte Fehlinterpretation verhindert ist. Eine möglicherweise resultierende Umschaltung der Betriebsart folgt dann im Zeitpunkt $T_V$, zu Beginn der Refraktärzeit des Ventrikels. Eine Störerkennung ist damit auch grundsätzlich während der Refraktärzeiten mit Ausnahme der Austastzeiten möglich.

Die in Figur 3a wiedergegebene strichpunktierte Linie deutet (entsprechend der Darstellung in Figur 1) an, dass der Zähler 311 bei einem Betrieb mit teilweise zur Energieersparnis in einen Wartezustand setzbaren Schaltungsteilen permanent in Betrieb bleibt, während die rechts von der strichpunktierten Linie befindlichen Elemente lediglich bedarfsweise aktiviert werden, wenn nämlich der Ausgang des Zählers ein Signal an das ODER-Gatter 30 in Figur 1 ausgibt, welches eine Beendigung des Wartezustands der übrigen Schaltglieder auslöst. Hiermit wird also ein Beispiel für einen Betrieb gegeben, bei dem ein intern im Schrittmacher erzeugtes Zeitsignal, welches den Ablauf einer Zeitspanne markiert, in der ein erwartetes Ereignis nicht eingetreten ist, die Signalverarbeitungsmittel entsprechend diesem Ergebnis verändert.

Bei der in Fig. 3b dargestellten Ausführung der zur Feststellung des Tachykardiezustands vorgesehenen Logik entsprechen die Bauelemente 331 bis 337 den mit 311 bis 317 bezeichneten in Fig. 3a, wobei die Bezifferung in derselben Folge vorgenommen worden ist. Der Zähler 331 zählt jedoch bis zu einer Zahl p, welche so gewählt ist, dass eine Impulsfolgezeit definiert wird, welche die Grenze zu einer Impulsrate im Atrium bildet, die einen Tachykardie-Zustand kennzeichnet. Die genannte Folgezeit beträgt dabei 350 ms.

Der Rücksetzeingang des Flip-Flops 333 ist jedoch nicht direkt mit dem Ausgang des UND-Gatters 332 verbunden, sondern es ist hier ein ODER-Gatter 338 zwischengeschaltet, dessen weiterer Eingang mit dem Ausgang eines UND-Gatters 339 verbunden ist, dessen einer Eingang wiederum an den Ausgang des Flip-Flops 333 angeschlossen ist. Der andere Eingang des UND-Gatters 339 ist an den Ausgang der Austast-Schaltung 3 angeschlossen und erhält ein Signal, wenn auf einen Stimulationsimpuls hin die Atriumeingangsstufe 101 für Signale gesperrt wird. Damit wird erreicht, dass ein Rücksetzen des Flip-Flops 333 jeweils dann vorgenommen wird, wenn ein Stimulationsimpuls abgegeben wurde und eine Austastung der Atriumseingangsstufe 101 erfolgte. Auf diese Weise ist sichergestellt, dass das den Tachykardie-Zustand kennzeichnende Flip-Flop 335 stets erst dann zurückgesetzt wird, wenn nach einer Austastzeit der volle Zeitraum durchlaufen wurde, welcher zwischen zwei aufgenommenen Impulsen im Atrium liegen muss, um das Ende einer Tachykardie festzustellen. Damit können nicht durch die vorzeitige falsche Entscheidung der Annahme der Beendigung des Vorliegens einer Tachykardie Stimulationsimpulse zu Zeiten abgegeben werden, welche eine Gefährdung des Patienten bedeuten können.

Durch die gewählte Übernahme im Zeitpunkt $T_A$ über die weiteren Logikmittel (Betriebsartspeicher 4) ist weiterhin sichergestellt, dass die Veränderung des Betriebsverhaltens ebenfalls zu einem Zeitpunkt und in einer Weise erfolgt, welche eine optimale Synchronität mit dem Herzverhalten des Patienten sicherstellen.

Die von der Refraktärzeitsteuerung getrennte Ermittlung der Störungs- und Tachykardiezustände im Vorhof gewährleistet, dass beide Signalzustände optimal erfasst werden, wobei durch das Zurücksetzen des Tachykardiezählers allein während der Austastzeiten sichergestellt ist, dass die Störerkennung unabhängig davon fortgesetzt wird und nicht durch ein Zurücksetzen und die daraus resultierende Bildung eines verkürzten Messzeitintervalls für die Störerkennung im Zusammenhang mit den Austast-Impulsen ein Störsignal vorgetäuscht wird.

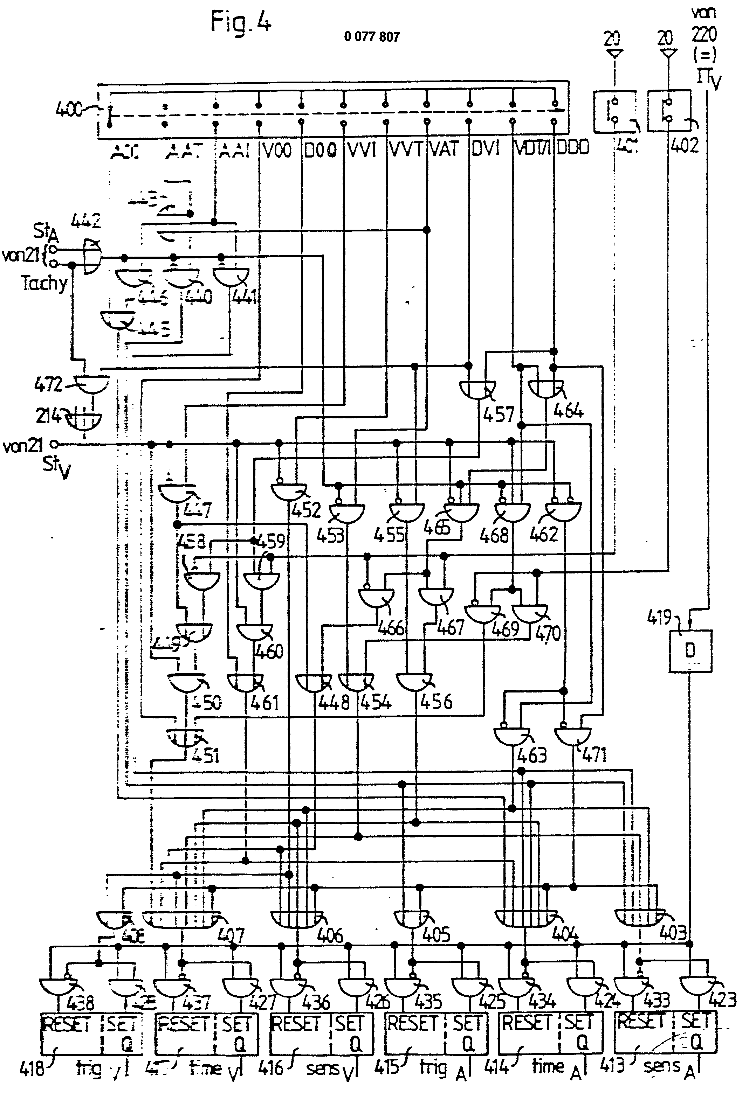

In Figur 4 sind diejenigen Bauelemente dargestellt, welche einerseits eine Programmierung vorgegebener Betriebsarten durch äussere Schaltmittel ermöglichen und weiterhin die Umschaltung der Betriebsart im Falle von an den Eingängen A und V einfallenden Störimpulsen bzw. im Falle einer Tachykardie im Vorhof bewirken. Insbesondere hervorzuheben ist dabei die Möglichkeit, den im Falle des Eintretens eines oder mehrerer der vorgenannten Ereignisse einzuschaltenden Betriebsart aus einer oder mehreren für diesen Fall festgelegten Betriebsarten auszuwählen und diese Auswahl vorher mittels Programmierung festzulegen. Bei der in Figur 4 dargestellten Schaltungsanordnung handelt es sich um diejenigen Baugruppen, die in der Blockschaltung gemäss Figur 1 als Steuerschaltung für die Betriebsarten in einem Element zusammengefasst sind.

Durch das über das Schaltelement 20 zu dem Block 4 gelangende Programmiersignal wird einerseits mittels eines Schaltelementes 400 eine (permanente) Betriebsart des Schrittmachers ausgewählt, die dann gültig ist, wenn keine Störsignale aufgenommen werden und auch der Tachykardiezustand nicht vorhanden ist. Durch die Programmierung ist einer der Betriebsarten auswählbar. Zusätzlich kann – ebenfalls durch die vorgenommene Programmierung – durch weitere Schaltelemente 401 und 402 festgelegt werden, welcher von zwei möglicherweise zur Verfügung stehenden alternativen Betriebsarten im Falle des Auftretens einer oder mehrerer der genannten Bedingungen in Funktion treten soll, wobei beim Auftreten verschiedener Störungen der Schalter 401 bezüglich einer ersten Art und der Schalter 402 bezüglich einer zweiten Art von Störungen setzbar ist. Die bei dem dargestellten Ausführungsbeispiel gewählte Zuordnung kann je nach den gestellten Anforderungen geändert, eingeschränkt oder erweitert werden. So ist auch die Festlegung durch die Schalter 401 und 402 in der dargestellten Form nicht zwingend, sondern kann durch zusätzliche Schaltelemente in der Weise verändert werden, dass für unterschiedliche Störungs- oder Tachykardiezustände verschiedene alternative Betriebsfunktionen aktiviert werden.

Über die Ausgangsleitungen des Schaltelementes 400, die mit den üblichen Bezeichnungen für mögliche Schrittmacher-Betriebsfunktionen gekennzeichnet sind, werden (unter vorläufiger Ausserachtlassung der für die Einschaltung der alternativen Betriebsfunktionen vorgesehenen Logik-Bauelemente) ODER-Gatter 403 bis 408 alternativ aktiviert, wobei jedem dieser ODER-Gatter 403 bis 408 ein Flip-Flop 413 bis 418 zugeordnet ist, an deren Ausgängen die das Betriebsverhalten des Schrittmachers bestimmenden Ausgangssignale, welche mit den Ausgangsleitungen des Blocks 4 in Figur 1 übereinstimmen, festgelegt werden.

Ein Setzen bzw. Zurücksetzen des Flip-Flops 413 im Falle einer Umprogrammierung durch das Signal $T_V$ erfolgt, wobei ein Verzögerungsglied 419 dafür sorgt, dass die für die Erkennung und Auswertung der die Betriebsart beeinflussenden Signale, die ebenfalls zum Zeitpunkt $T_V$ stattfindet, abgeschlossen sind, bevor eine Betriebsartänderung eintritt.

Kriterium für die Bemessung der Verzögerungszeit des Gliedes 419 ist dabei, dass die Umschaltung der Betriebsart des Schrittmachers innerhalb derjenigen Zeit abgeschlossen sein muss, während der beide Eingänge A und V refraktär sind. (Es gilt auch für die übrigen von einem zum Zeitpunkt $T_V$ erscheinenden Impuls ausgelösten Signale, dass innerhalb des gesamten dargestellten Schrittmacherkonzepts – je nach Ausbauzustand – gegebenenfalls durch zusätzliche Verzögerung eine derartige Staffelung der Signalverarbeitung erfolgt, dass solche Schritte erst dann eingeleitet werden, wenn die vorangehenden Verarbeitungen, die für diese Schritte Voraussetzung sind, abgeschlossen sind. Es gilt dabei die Reihenfolge: Beendigung der Verarbeitung für den vorangegangenen Herzzyklus – Betriebsart-/Programmänderung – Festlegung der weiteren Daten für den nächsten Zyklus.)

Die Umschaltung der Flip-Flops erfolgt über UND-Gatter 423 bis 428 bzw. 433 bis 438, wobei je nach dem am Ausgang der ODER-Gatter 403 bis 408 anliegenden Signalzustände eines der beiden mit dem Setz- bzw. Rücksetzeingang der Flip-Flops 413 bis 418 verbundenen UND-Gatter aktiviert wird, wenn vom Ausgang der Schaltung 419 der gegenüber der Zeit $T_V$ verzögerte Impuls $I_{TV}$ erscheint. Die Ausgänge der ODER-Gatter 403 bis 408 sind zu diesem Zweck jeweils mit einem nichtinvertierenden Eingang eines der UND-Gatter 423 bis 428 und einem invertierenden Eingang der UND-Gatter 433 bis 438 verbunden.

Die Funktionen der Flip-Flops 413 bis 418 sind die folgenden:

Flip-Flop 413: Signalaufnahme im Atrium

Flip-Flop 414: Zeitgesteuerte Auslösung von Stimulationsimpulsen im Atrium

Flip-Flop 415: Durch herzeigene Signale getriggerte Auslösung von Stimulationsimpulsen im Atrium

Flip-Flop 416: Signalaufnahme im Ventrikel

Flip-Flop 417: Zeitgesteuerte Auslösung von Stimulationsimpulsen im Ventrikel

Flip-Flop 418: Durch herzeigene Signale getriggerte Auslösung von Stimulationsimpulsen im Ventrikel

Es ist ersichtlich, dass aufgrund der bisher dargestellten Logikmittel in der Betriebsart «A00» durch Setzen des Flip-Flops 414 das zeitgesteuerte Auslösen von Atriumimpulsen festgelegt ist. Eine Signalaufnahme aus Atrium oder Ventrikel erfolgt nicht und infolgedessen ist auch keine Umschaltung bei hier auftretenden Störsignalen erforderlich.

Entsprechendes gilt für die Betriebsarten «V00» und «D00», wobei hierbei noch das Flip-Flop 417 alternativ oder zusätzlich aktiviert ist.

Die Betriebsarten «AAT» und «AAI» aktivieren bei ungestörtem Atrium und, wenn keine Tachykardie vorhanden ist, über UND-Gatter 440 bzw. 441, deren invertierende Eingänge gemeinsam mit dem Ausgang eines ODER-Gatters 442 verbunden sind, an dessen Eingänge diese Zustände anzeigende Signale gelangen, wenn im Atrium eine Störung auftritt bzw. der Tachykardiezustand ermittelt wurde, die entsprechenden der Flip-Flops 413 bis 415. Ist am Ausgang des ODER-Gatters 442 ein Signal mit dem logischen H-Zustand vorhanden, so wird bei aktiviertem «AAT»- oder «AAI»-Zustand über ein ODER-Gatter 443 und ein UND-Gatter 446 sowie das ODER-Gatter 445 der «A00»-Zustand eingestellt, wie er auch direkt durch eine entsprechende Programmierung gewählt werden kann.

Durch Aktivierung der Betriebsarten «V00» bzw. «D00» werden diese eingeschaltet, ohne dass eine Betriebsartsänderung durch über die Elektroden aufgenommenen Signale möglich wäre. Die Betriebsart «VVI» wird aktiviert, wenn dieser Zustand über den Programmschalter 400 gesetzt ist und keine Störungen im Ventrikel vorliegen, so dass ein Ausgangssignal vom UND-Gatter 447 an das ODER-Gatter 448 gelangt. Liegt eine Störung im Ventrikel vor, so geht der Schrittmacher durch die Verknüpfung der entsprechenden Signale über ein ODER-Gatter 449, ein UND-Gatter 450 sowie ein weiteres ODER-Gatter 451 in den Betriebszustand «V00» über.

Die Betriebsart «VAT» ist bei ungestörtem Ventrikel (kein Signal am invertierenden Eingang des UND-Gatters 452) durch entsprechende Programmierung einstellbar.

In der Betriebsart «VAT» wird über das UND-Gatter 453 ein ODER-Gatter 454 aktiviert, wenn das UND-Gatter 453 nicht durch das Ausgangssignal des ODER-Gatters 442 über seinen invertierenden Eingang gesperrt ist. Im Falle einer Störung im Atrium oder des Vorliegens von Tachykardie erfolgt eine Umschaltung in den Zustand «V00», was durch eine zum ODER-Gatter führende Leitung bewirkt wird, wobei die Umschaltbedingungen denjenigen in den Modes «AAT» bzw. «AAI» entsprechen.

In der Betriebsart «DVI» wird bei ungestörtem Ventrikeleingang (kein Signal am invertierenden Eingang eines UND-Gatters 455), dessen Ausgangssignal zu einem ODER-Gatter 456 geleitet, welches die dieser Betriebsart entsprechenden Aktivierungen auslöst.

Bei gestörtem Ventrikel gelangt das Signal über ein ODER-Gatter 457 an die Eingänge zweier UND-Gatter 458 und 459, deren weitere Eingänge (wobei der weitere Eingang beim UND-Gatter 458 invertierend ist) mit dem Ausgang des Schalters 401 verbunden sind. Je nach dem, ob dieser durch Programmierung gesetzt (Verbindung mit logischem H-Pegel) oder nicht gesetzt (entsprechend dem logischen L-Pegel) ist, wird eines der UND-Gatter 458 bzw. 459 durchgeschaltet und aktiviert über das UND-Gatter 450 im Falle einer Störung im Ventrikel über das ODER-Gatter 451 den Zustand «V00» oder über UND-Gatter 460, dessen weiterer Eingang entsprechend mit der bei gestörtem Ventrikel ein logisches «H-Signal» führenden Leitung verbunden ist, über ein ODER-Gatter 461 den Zustand «D00». Damit kann von ausserhalb des Schrittmachers her für den Fall eines im Ventrikel aufgenommenen Störsignals durch eine entsprechende Voreinstellung festgelegt werden, welchen Betriebszustand der Schrittmacher in diesem Fall einnehmen wird. Somit besteht eine zusätzliche therapeutische Möglichkeit in der Art, dass der Arzt für den Fall, dass ein ursprünglich von ihm gewählter Betriebszustand des Schrittmachers nicht beibehalten werden kann, einen Alternativzustand auswählen kann, der sich im Fall des betreffenden Patienten als besonders günstig darstellt.

Eine entsprechende Wahlmöglichkeit ist auch beim Betriebszustand «VDT/I» vorgesehen, wobei das Fehlen von Störungen im Atrium und Ventrikel und auch das Nichtvorliegen des Tachykardie-Zustands mittels eines UND-Gatters 462 ermittelt wird, dessen beide invertierende Eingänge einerseits mit dem Ausgang des ODER-Gatters 442 bzw. mit der eine Störung im Ventrikel anzeigenden Leitung verbunden sind. Der Ausgang des UND-Gatters 462 führt zu einem UND-Gatter 463, dessen Ausgangssignal die für den Betriebszustand «VDT/I» notwendigen logischen Verknüpfungen auslöst. Im Falle einer Störung im Atrium allein wird über ein ODER-Gatter 464 und ein UND-Gatter 465 bei Vorliegen einer Störung im Atrium bzw. Tachykardie und Anwesenheit einer Störung im Ventrikel je nach Stellung des Schalters 401 über UND-Schalter 466 bzw. 467 entweder der Zustand «VVI» oder der Zustand «DVI» aktiviert.

Ist dagegen der Ventrikel gestört, während der Sinusrhythmus regulär aufgenommen wird, gelangt über ein diese Signalzustände auswertendes UND-Gatter 468 in Abhängigkeit von der Stellung des Schalters 402 ein Signal zu UND-Gattern 469 und 470 alternativ zu den ODER-Gattern 451 bzw. 454, so dass in diesem Fall alternativ die Betriebszustände «V00» oder «VAT» aktivierbar sind. Bei gestörtem Ventrikeleingangssignal bleibt demnach dem behandelnden Arzt die Möglichkeit, entweder im Atrium auftretende Signale noch zur Triggerung der Ventrikel-Stimulation heranzuziehen oder aber den Ventrikel starrfrequent zu stimulieren. Der Betriebszustand «VDT/I» geht damit beispielsweise in den «VAT»-Zustand über.

Der Fall des ungestörten Betriebs im Mode «DDD» wird entsprechend der Funktion des UND-Gatters 463 für den Fall «VDT/I» über ein UND-Gatter 471 signalisiert.

Bei gestörtem Atriumeingang bzw. vorliegender Tachykardie werden die Umschaltungen entsprechend wie im vorgenannten Betriebszustand über das ODER-Gatter 464 ausgelöst, während bei gestörtem Ventrikel ebenfalls eine entsprechende Verknüpfung über das ODER-Gatter 457 herbeigeführt wird.

Es ist ersichtlich, dass die vorgenannten Mode-Umschaltungen und die Möglichkeit der Vorprogrammierung alternativer Modes im Falle von Störungen der Signaleingänge oder Tachykardie auf unterschiedliche Weise vorgegeben werden können, wobei insbesondere für die Terminierung von Tachykardien auch Sonderbetriebsweisen aktiviert werden können, die an dieser Stelle nicht detailliert beschrieben zu werden brauchen, da sie dem Fachmann aus entsprechenden Veröffentlichungen bekannt sind.

Die Möglichkeit, mit einer Mode-Änderung auch Betriebsparameter zu verändern, ist für den Fall des DVI-modes durch die Verbindung vom Ausgang «DVI» des Schalters 400 zu einem Eingang des ODER-Gatters 214 über ein UND-Gatter 472 wiedergegeben. Bei Aktivierung dieses UND-Gatters 472 im Falle von Tachycardie (Leitung zum Eingang «Tachy») beim «DVI»-Betrieb erfolgt eine zwangsläufige Verkürzung der programmierten AV-Überleitungszeit ($T_0$ bis $T_V$) auf einen unkritischen Wert, der für diese Ausführung als in Block 216 enthalten angenommen werden soll.

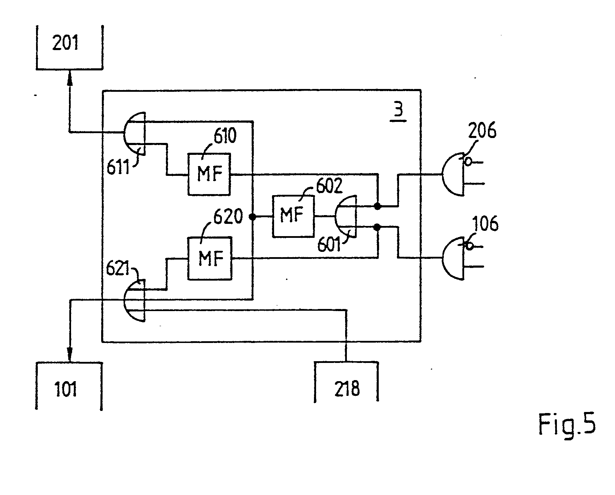

In Figur 5 ist die Austaststufe 3 gemäss Figur 1 in Einzelheiten dargestellt. Die Ausgangssignale der UND-Gatter 106 und 206 gelangen an die Eingänge eines ODER-Gatters 601, dessen Ausgangssignal eine Monoflop-Schaltung 602 aktiviert, welche für 25 ms einen Ausgangsimpuls abgibt. Dieser Impuls gelangt über ODER-Gatter 611 bzw. 621 sowohl an die Eingangsstufe 101 für das Atrium als auch an die Eingangsstufe 201 für den Ventrikel und macht beide für diesen Zeitraum für eingehende Signale undurchlässig. Die Sperrung beider Eingangsstufen für 25 ms erfolgt also unabhängig davon, ob an das Atrium oder an den Ventrikel ein Stimulationsimpuls abgegeben wurde.

Die dargestellte Schaltung weist darüber hinaus Mittel auf, welche es gestatten, zwischen der stimulierten und der nicht stimulierten Kammer bezüglich der Länge der Austastzeit zu unterscheiden. Dabei wird bei der dargestellten Anordnung die Austastzeit für die stimulierte Kammer erhöht, um sicherzustellen, dass die Polarisationseffekte an der Elektrode abgeklungen sind, bevor eine erneute Signalaufnahme erfolgen kann. Zu diesem Zweck sind zwei jeweils einer Kammer zugeordnete Monoflop-Schaltungen 610 und 620 vorgesehen, welche auf einen Eingangsimpuls hin einen Ausgangsimpuls abgeben, welcher der verlängerten Austastdauer für die stimulierte Kammer – bei dem dargestellten Ausführungsbeispiel 60 ms – entspricht. Die Eingänge der Monoflop-Schaltungen sind dabei direkt mit den Ausgängen der UND-Gatter 106 bzw. 206 verbunden, während die Austastimpulse jeweils den zweiten Eingängen der ODER-Gatter 611 und 621 zugeführt werden, wobei im Falle der Stimulation einer der Kammern sich die Aktivierungszeiten der Stufen 602 und 620 bzw. 602 und 610 am Ausgang der ODER-Gatter 611 bzw. 621 überlagern.

Durch die Verbindungsleitung vom Monoflop 218 (in Figur 1 enthalten) zu einem weiteren Eingang des ODER-Gatters 621 tritt bei einer Refraktärzeitverlängerung im Atrium nach einer Extrasystole im Ventrikel der Eingangsverstärker auch für die Störsignal und Tachykardieerkennung gesperrt.

## Patentansprüche

1. Herzschrittmacher, der Mittel zum Festhalten eines dem Vorliegen eines Tachykardiezustandes zugeordneten Signales aufweist, das erzeugt wird, wenn mehrere aufeinanderfolgende Aktionen des Atriums einen vorgegebenen Zeitabstand unterschreiten sowie weitere Schaltmittel, welche die erstgenannten Mittel in einen Zustand ohne Signalausgabe zurücksetzen, dadurch gekennzeichnet, dass das dem Vorliegen des Tachykardiezustands zugeordnete Signal erzeugt wird, wenn zwei aufeinanderfolgende Aktionen ($T_A$) im Atrium den vorgegebenen Zeitabstand unterschreiten sowie ferner Mittel (3) zum Austasten (Sperrung für Eingangssignale) einer Eingangsschaltung (101) für vom Atrium aufgenommene Signale vorgesehen sind, die bei Abgabe eines Stimulationsimpulses ($T_A$ oder $T_V$) einen Austastimpuls ($T_{blankA}$) an die Eingangsschaltung abgeben, und dass die Zurücksetzung in den Zustand ohne Signalausgabe erfolgt, wenn seit dem letzten aufgetretenen Austastimpuls mindestens ein Zeitraum vergangen ist, welcher grösser ist, als der vorgegebene Zeitabstand, ohne dass vom Atrium ein Signal aufgenommen wurde.

2. Herzschrittmacher nach Anspruch 1, dadurch gekennzeichnet, dass die Mittel zum Festhalten des Tachykardiezustands (103) Zeitmessmittel (331) zum Ermitteln des Unterschreitens des vorgegebenen Zeitabstands aufweisen, die durch das Erscheinen eines im Atrium aufgenommenen Signals ($T_A$) jeweils in einen Ausgangszustand zurückgesetzt werden.

3. Herzschrittmacher nach Anspruch 2, dadurch gekennzeichnet, dass die Ermittlung des Zeitabstands jeweils zwischen einem Austastimpuls ($T_{blankV}$) aufgrund einer Stimulation im Ventrikel ($T_V$) und dem Zeitpunkt, zu dem eine Stimulation im Atrium erfolgen kann, bzw. dem Beginn des mit der Stimulation einhergehenden Austastimpulses ($T_{blank}$), erfolgt.

4. Herzschrittmacher nach Anspruch 2 oder 3, dadurch gekennzeichnet, dass bei Überschreiten des vorgegebenen Zeitabstands durch die Zeitmessmittel (331) das Nichtvorhandensein einer Tachykardie ausgegeben wird und das Zurücksetzen in den Zustand ohne Signalausgabe auf das die Eingangsschaltung für im Atrium aufzunehmende Signale austastende Signal hin erfolgt.

5. Herzschrittmacher nach Anspruch 4, dadurch gekennzeichnet, dass die Übernahme des die Beendigung der Tachykardie anzeigenden Signals in einen Betriebsartenspeicher (4) nach einer vom Ventrikel herrührenden Herzaktion bzw. nach Erreichen des Zeitpunkts, zu dem ein Stimulationsimpuls an den Ventrikel abzugeben ist, auch erfolgt, während der Signaleingang bzw. die Signaleingänge für vom Herzen abgeleitete Signale zur sonstigen zeitlichen Synchronisation für Eingangssignale gesperrt sind.

6. Herzschrittmacher nach einem der vorangehenden Ansprüche, dadurch gekennzeichnet, dass für die Erkennung von Tachykardiezuständen und für die Erkennung von Störsignalen in Atrium und/oder Ventrikel einander entsprechende Anordnungen verwendet werden.

7. Herzschrittmacher nach Anspruch 6, dadurch gekennzeichnet, dass die Anordnungen getrennt von den übrigen Signalverarbeitungsschaltungen aufgebaut sind und jeweils einen separaten Signalspeicher (331, 333) zum Festhalten des jeweiligen Signalzustands aufweisen.

## Claims

1. A cardiac pacemaker which comprises means for maintaining a signal which is associated with the presence of a state of tachycardia and which is produced if a plurality of successive actions of the atrium fall short of a preset interval of time, as well as further circuit elements which reset the first-mentioned means into a state without signal output, wherein the signal associated with the presence of the state of tachycardia is generated if two successive actions ($T_A$) in the atrium fall short of the preset interval of time and further means (3) are provided for blanking (blocking to input signals) an input circuit (101) for signals picked up from the atrium, which means, on delivery of a stimulation pulse ($T_A$ or $T_V$), deliver a blanking pulse ($T_{blankA}$) to the input circuit, and wherein the resetting into the state without signal output is effected if a period of time has elapsed since the last blanking pulse appeared which is greater than the predetermined interval of time, without a signal having been picked up from the atrium.

2. A cardiac pacemaker as claimed in Claim 1, wherein the means for maintaining the state of tachycardia (103) comprise time-measuring means (331) to detect the falling short of the preset interval of time, which means are reset to an initial state each time by the appearance of a signal ($T_A$) picked up in the atrium.

3. A cardiac pacemaker as claimed in Claim 2, wherein the determination of the interval of time is effected each time between a blanking pulse ($T_{blankV}$) as a result of a stimulation in the ventricle ($T_V$) and the moment at which a stimulation in the atrium can take place or the beginning of the blanking pulse ($T_{blank}$) accompanying the stimulation.

4. A cardiac pacemaker as claimed in Claim 2 or 3, wherein if the preset interval of time is exceeded, the absence of a state of tachycardia is signalled by the time-measuring means (331) and the resetting into the state without signal output is effected in response to the signal blanking the input circuit to signals to be picked up in the atrium.

5. A cardiac pacemaker as claimed in Claim 4, wherein the transfer of the signal indicating the end of the tachycardia into an operating mode store (4), after a heart action originating from the ventricle or after the moment is reached when a stimulation pulse should be delivered to the ventricle, is effected also while the signal input or the signal inputs for signals derived from the heart for other synchronization in time are blocked to input signals.

6. A cardiac pacemaker as claimed in one of the preceding Claims, wherein devices corresponding to one another are used for the recognition of states of tachycardia and for the recognition of spurious signals in the atrium and/or ventricle.

7. A cardiac pacemaker as claimed in Claim 6, wherein the devices are constructed separately from the other signal processing circuits and each have a separate signal store (331, 333) to retain the particular signal state.

**Revendications**

1. Stimulateur cardiaque possédant des moyens pour retenir un signal coordonné à l'existence d'un état de tachycardie, signal qui est produit lorsque la durée de plusieurs actions consécutives de l'oreillette descend au-dessous d'un intervalle de temps préfixé, ainsi que d'autres moyens de commutation qui ramènent les moyens mentionnés en premier à un état de non-délivrance de signaux, caractérisé en ce que le signal coordonné à l'existence de l'état de tachycardie est produit lorsque la durée de deux actions ($T_A$) consécutives dans l'oreillette descend au-dessous de l'intervalle de temps prédéterminé, que des moyens de suppression (3) sont prévus, en outre, pour bloquer les signaux d'entrée d'un circuit d'entrée (101) pour ce qui concerne des signaux recueillis de l'oreillette, moyens qui, en cas de délivrance d'une impulsion de stimulation ($T_A$ ou $T_V$), délivrent une impulsion de suppression ($T_{blankA}$) au circuit d'entrée, et que la remise à l'état de non-délivrance de signaux a lieu lorsque, depuis la dernière impulsion de suppression, il s'est écoulé un laps de temps, sans qu'un signal a été recueilli de l'oreillette, qui est au moins plus long que l'intervalle de temps préfixé.

2. Stimulateur selon la revendication 1, caractérisé en ce que les moyens pour retenir l'état de tachycardie (103) comportent des moyens de mesure du temps (331) destinés à déterminer la diminution de ladite durée au-dessous de l'intervalle de temps prédéterminé et qui sont remis chaque fois à un état initial par l'apparition d'un signal ($T_A$) recueilli dans l'oreillette.

3. Stimulateur selon la revendication 2, caractérisé en ce que la détermination dudit intervalle de temps s'effectue chaque fois entre une impulsion de suppression ($T_{blankV}$), produite en raison d'une stimulation dans le ventricule ($T_V$), et le moment où une stimulation dans l'oreillette peut avoir lieu, respectivement le début de l'impulsion de suppression ($T_{blank}$) qui accompagne le début de la stimulation.

4. Stimulateur selon la revendication 2 ou 3, caractérisé en ce que, en cas de dépassement de l'intervalle de temps préfixé par les moyens de mesure du temps (331), un signal de non-existence d'une tachycardie est délivré et la remise à l'état de non-délivrance des signaux s'effectue à la suite du signal de suppression rendant le circuit d'entrée inactif pour des signaux à recueillir dans l'oreillette.

5. Stimulateur selon la revendication 4, caractérisé en ce que la reprise du signal indiquant la fin de la tachycardie dans une mémoire (4) de modes de fonctionnement, à la suite d'une action du cœur issue du ventricule, ou après que l'instant où une impulsion de stimulation est à délivrer au ventricule est atteint, s'effectue également pendant que l'entrée de signal ou les entrées de signal pour des signaux dérivés du cœur, sont bloquées pour des signaux d'entrée à des fins de synchronisation dans le temps en dehors de ce processus.

6. Stimulateur selon une des revendications précédentes, caractérisé en ce qu'il utilise des dispositifs qui correspondent entre eux pour la détection d'états de tachycardie et pour la détection de signaux parasites dans l'oreillette et/ou le ventricule.

7. Stimulateur selon la revendication 6, caractérisé en ce que les dispositifs sont constitués séparément des autres circuits de traitement de signaux et présentent chacun une mémoire de signal séparée (331, 333) pour retenir chaque fois l'état de signal correspondant.

0 077 807

Fig. 1

Fig. 2

Fig.3a

Fig.3b

Fig.4

0 077 807

21

Fig.5